(19)

Europäisches
Patentamt
European
Patent Office
Office européen
des brevets

(11) **EP 3 326 662 A1**

(12) **EUROPEAN PATENT APPLICATION**
published in accordance with Art. 153(4) EPC

(43) Date of publication:
**30.05.2018 Bulletin 2018/22**

(21) Application number: **16848016.8**

(22) Date of filing: **08.09.2016**

(51) Int Cl.:
**A61L 27/56** (2006.01)  **A61L 27/04** (2006.01)
**A61L 27/08** (2006.01)

(86) International application number:
**PCT/CN2016/098414**

(87) International publication number:
**WO 2017/050134 (30.03.2017 Gazette 2017/13)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **21.09.2015  CN 201510600351**

(71) Applicant: **Chongqing Runze Pharmaceutical Co. Ltd.**
**Chongqing 400042 (CN)**

(72) Inventor: **YE, Lei**
**Chongqing 400042 (CN)**

(74) Representative: **Inal, Aysegul Seda**
**Yalciner Patent and Consulting Ltd.**
**Tunus Cad. 85/4**
**06680 Kavaklidere, Ankara (TR)**

(54) **USE OF POROUS MATERIAL**

(57)  The present invention relates to a new application of a porous material. The porous material is composed of pore cavities and cavity walls surrounding the pore cavities, wherein the pore cavities of the porous material are three-dimensionally interconnected; the capillary force of the porous material is 5 Pa or more; and a contact angle between a surface of the cavity wall of the porous material and a liquid phase material circulating therein is less than 90°. The porous material is applied as a microcirculation power source. The porous material is used in a circulation system as a microcirculation power source for providing material exchange. The porous material is used in a separation system as a microcirculation power source for providing material separation and movement. The porous material is used in a medical implant system as a microcirculation power source for providing tissue cell growth.

Fig.1

EP 3 326 662 A1

**Description**

**Technical field**

The present invention relates to a porous material, particularly to a new application of the porous material.

**Background**

As a kind of multi-functional engineering material with excellent performance, porous materials are widely used in many fields such as aerospace, atomic energy, electrochemical, petrochemical, metallurgy, machinery, medicine, environmental protection and construction industry for separation, filtration, gas distribution, catalysis, electrochemical processes, sound attenuation, shock absorption, shielding, heat exchange, implantation and other processes, because of the excellent, both, functional & structural physical and mechanical properties. For example, depending on the pores size of the porous materials, they can be used capture foreign materials in the fluid mediums, the filtration and separation of gases or liquids can be performed, so as to achieve media purification and separation. The porous metal can be used for absorbing energy, noise reduction, such as anti-punch for automotive applications, as a filter; porous metal can be used in heat exchangers, with high efficiency. Various high-performance fluid distribution elements of fluidized bed technology equipment in the oil industry, chemical industry and metallurgical industries, requires uniform pore distribution, high permeability, corrosion resistance, high temperature resistance, thermal shock resistance, and sufficient bearing strength. Porous metal can meet these comprehensive technical indicators. Porous metal has an excellent conductive performance, and has some self-supporting ability and large surface area to provide a broad interface electrochemical charge transfer space, and thus can be used as an excellent electrode material for a variety of batteries, fuel cells and solar cells. Porous metal can be used as a variety of structural materials such as the lightweight rigid sandwich panel prepared by aluminum foam which is used to reduce the mass of the automobile, and support body of the aircraft wing made by foam metal and so on. Porous metals can be used as flame arrester for preventing the spread of flames. Porous materials such as porous titanium, porous tantalum, porous hydroxyapatite, etc. can be used as biological materials, such as artificial bone, teeth, etc. Porous ceramics can be used as a catalyst carrier to promote the reaction. Porous ceramics can be made into photocatalyst carrier. Nano-titanium dioxide particles coated on the porous ceramic have a strong degradation characteristic of photocatalytic oxidation after UV excitation, which can catalyze organic and microorganism, so that the air is purified. Porous ceramics can also be used as temperature, humidity, gas, chemical or other sensors. The rigid polyurethane foam can be used as thermal insulation material, widely used in building energy conservation. The foam separation and enrichment technology is widely used in analytical chemistry, so far, there are 60 kinds of elements that can be separated and enriched by foam plastics. The perforated polytetrafluoroethylene fabric which is air permeable and waterproof can be used to make micro-porous waterproof and high quality sportswear, and similar foam cups can be used for artificial skin. Due to the low density of the polymeric foam, the polymeric foam has an extremely low unit volume loss factor, and is suitable for antenna housing and radio transmission housing.

Although the porous materials are widely used, insufficient attention has been paid to the capillary action of porous materials. Patent CN1636876A describes a method of enhancing water evaporation and chromatographic separation using a porous material having capillary action. One or more porous materials having capillary action are placed in salt pan, brackish pools or industrial wastewater or sewage pools containing non-volatile contaminants, such as natural fibers, synthetic fibers, metal fibers, carbon fiber cloths, plate-like porous ceramics, or plate-like diatomite and other substances, by using the porous material with capillary action to expand the evaporation area of water, strengthen the evaporation of water, increase the output of salt or handling capacities of the industrial wastewater or sewage pools containing non-volatile contaminants, saving energy and improving the separation efficiency. Although this patent mentions the capillary action of porous materials, no study has been conducted on the size of the capillary force and its correlation of infiltration with the cavity wall surfaces of the pores of the porous material and the liquid phase material flowing therein. Further, the degree of capillary action of the porous material is not clear. So far, it has never seen the application of porous materials as a microcirculation power source.

**Summary of the invention**

The present invention is to provide a porous material having a strong capillary force and an excellent infiltration with the transmitted liquid substance, which can be used as an application of microcirculation power source.

The inventor found that neither all porous materials have capillary force, nor all porous materials having the capillary force have sufficient capillary force. Only when the porous materials having a certain capillary force and an infiltration can be used as microcirculation power source.

For the porous material with capillaries, its capillary force must be strong enough, so that the effect will be significant and efficiency will be high. However, the capillary force of the porous material depends on many factors such as the solid material itself, the solid surface condition and the connectivity of pore penetration and pore size. Therefore, it must match some of the material's relevant factors to achieve a larger value for using as a power source.

The objective of the present invention is realized by the following technical solution:

A porous material, characterized in that: the porous material comprises pore cavities and cavity walls surrounding to form the pore cavities, wherein the pore cavities of the porous material are three-dimensionally interconnected; the capillary force of the porous material is 5 Pa or more; and a contact angle between a surface of the cavity wall of the porous material and a liquid phase material circulating therein is less than 90 °; the porous material is applied as a microcirculation power source.

Preferably, the porous material is used in a circulation system as a microcirculation power source for providing the power source of material exchange.

Preferably, the porous material is used in a separation system as a microcirculation power source for providing the power source of material separation and movement.

Preferably, the porous material is used in a medical implant system as a microcirculation power source for providing the power source of the growth of tissue cell.

The advantages of the present invention are as below:

1. The porous material according to the present invention has a capillary force of 5 Pa or more and has good infiltration of the liquid phase material intervening therein, thus the porous material has a large adsorption on the liquid phase material, so that it can be used as a microcirculation power source. In the circulation system, the porous material can be used as a microcirculation power source to provide power source for system material exchange. In the separation system, the porous material can be used as a microcirculation power source for providing power source of material separation and movement.

2. When the porous material of the present invention is used as a medical implant material, the capillary force of 5 Pa or more can accelerate the blood flow, and in particular, the porous material has good infiltration to the biological tissue fluid and becomes the microcirculation power source of biological tissue fluid and blood, to improve the poor microcirculation caused by lesions, repair and improve microcirculation, which is conducive to the recovery of the lesions.

3. When the contact angle between the surface of the cavity walls of the porous material according to the present invention and the liquid phase material circulated therein is less than 90°, the liquid phase material circulating therein will have good infiltration to the porous material and be good to the liquid phase material flowing in the porous material, which is conductive to help to increase the capillary force.

## Brief description of the drawings

The present invention will be further described with the accompanying drawings and embodiments.

Figure 1 is a schematic diagram of the capillary force test device designed based on GB5250-93.
Figure 2 is a schematic diagram of experiment of the microcirculation formed by the capillary force conformation of the porous material of the present invention.

## Detailed description of the invention

The detailed embodiments are given on the premise of the technical solutions of the present invention, but the protection scope of the present invention is not limited to the following embodiments. It will be apparent to those skilled in the art that various changes and modifications may be made without departing from the scope of the present invention as defined by the appended claims, according to the common knowledge and/or common means in the field, and should be included in the scope of the present invention.

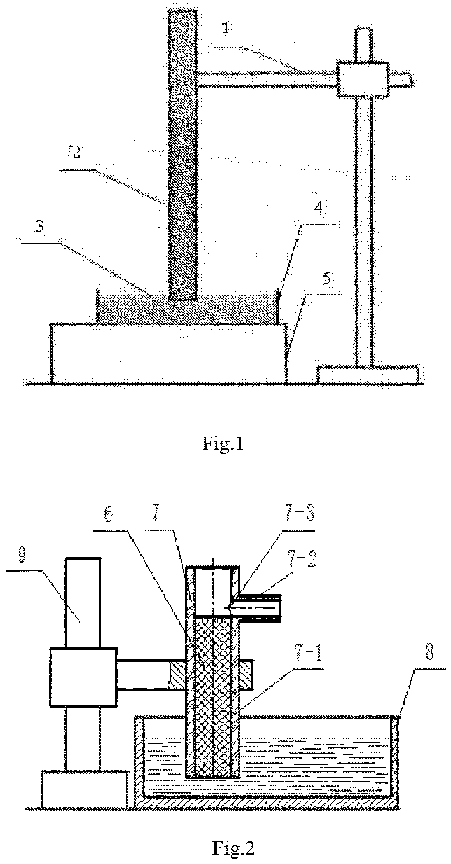

In FIG. 1, 1 is a clamping device, 2 is a porous material, 3 is a liquid phase material, 4 is a container for the liquid phase material 3, and 5 is an electronic balance.

According to the device shown in FIG.1, the capillary force of porous materials can be measured, the measuring processes are:

(1) The container 4 filled with the liquid phase material 3 is placed on the electronic balance 5 and the electronic balance 5 is reset;
(2) The porous material 2 is vertically fixed on the clamping device 1;
(3) By adjusting the clamping device 1, put the porous material 2 stretches into liquid level of the liquid phase material 3 for 1-2mm, and then start the timer to measure timing;
(4) When the value of the electronic balance begins to become negative, that is, the value is quality of liquid phase material which the porous material 2 sucks from the liquid phase material 3;
(5) According to the formula (2), calculating the capillary force $\Delta P$.

$$\Delta P = \mu M^2 / [2K\varepsilon \, (\rho S)^2 t] \hspace{3cm} (2)$$

Wherein, $\mu$ is the kinetic viscosity of the liquid phase material flowing through the porous material;
M is the quality of the liquid phase material sucked by the porous material;
K is the permeability of the porous material;
$\varepsilon$ is the porosity of the porous material;
$\rho$ is the density of the liquid phase material flowing through the porous material;
S is the cross-sectional area of the porous material;
t is the time that the liquid medium takes for rising.

In Fig. 2, 6 is a porous material, 7 is a plastic pipe, the plastic pipe 7 is divided into two parts, a main pipe 7-1 and a branch pipe 7-2. The main pipe 7-1 is connected with the branch pipe 7-2, 7-3 is a linking part. The outer diameter of the porous material 6 is made according to the inner diameter of the main pipe 7-1. The porous material 6 is put into the main pipe 7-1. 8 is a vessel, the liquid medium is contained in the vessel 8. 9 is a fixing seat for fixing the plastic pipe 7. The main pipe 7-1 of the plastic pipe 7 containing the porous material 6 is placed into the vessel 8. The porous material 6 is above a certain height in the liquid medium, the top of the porous material 6 is located below the lower portion of the link part 7-3. When the porous material 6 has a capillary force, the liquid medium contained in the vessel 8 will be sucked into the porous material. When the capillary force is strong enough, the liquid medium will rise to the top of the porous material, above the liquid level and reaches the link part 7-3, and flows out of the branch pipe 7-2, and then flows into the vessel 8 to form circulation.

**Embodiment 1**

The porous material of the present embodiment is porous silicon carbide, the pores of which are three-dimensionally interconnected with a porosity of 70% and an average pore diameter of 1260 $\mu$m. When the liquid phase material is deionized water, the capillary force at 20°C is 5.2Pa, which is calculated by using the device shown in FIG.1 and is calculated by the formula (2). Using the same materials and processes of preparation of the porous silicon carbide to prepare a plane with the same state as cavity wall of the porous silicon carbide. With the help of the JY-82 contact angle measuring instrument, using drop method to test with droplet volume of 2$\mu$l at the test environment temperature of 20 °C, the measured contact angle between the plane and deionized water is 88.7°. The material is tested with the device shown in FIG. 2 and the porous material 6 is 40 mm above the deionized water level. The test shows that the water quickly rises to the top of the porous material 6, flows out of the branch pipe 7-2, and then flows into the vessel 8 to form continuous cyclic process. This kind of material is used in the microcirculation system part of the small-scale water treatment circulation system for exchange of the water to be treated and the treated water. The application shows that, this kind of porous silicon carbide, because of its capillary force up to 5.2 Pa, with a strong suction force, which becomes one of the power sources, accelerates the flow of water relative to the absence of porous silicon carbide. Further, the test shows that the flow rate through the porous silicon carbide is increased by 21% relative to that without the porous silicon carbide, thereby increasing the exchange efficiency, but also increasing the exchange volume to achieve energy saving.

**Embodiment 2**

The porous material of the present embodiment is porous quartz, the pores of which are three-dimensionally interconnected with a porosity of 60% and an average pore diameter of 200 $\mu$m. When the liquid medium is kerosene, the porous quartz is tested at 20°C using the device shown in FIG. 1 and the formula (2) to calculate the capillary force up to 154Pa. Using the same materials and processes of the preparation of the porous quartz to prepare a plane with the same state as cavity walls of the porous quartz. According to the test method and conditions of Embodiment 1 to test, the contact angle between the plane and kerosene is 50°. The kerosene has good infiltration to such surface. The material is tested by the device shown in FIG. 2, and the porous material 6 is 50 mm above the kerosene level. The tests show that the kerosene quickly rises to the top of the porous material 6, flows out of the branch pipe 7-2, and then flows into the vessel 8 to form a continuous cyclic process. The material is used for kerosene filtration devices. Due to the capillary force of up to 154 Pa, with a strong suction force, it becomes one of the power sources. The power source accelerates the flow of kerosene containing solid impurity particles through the porous quartz, accelerating the separation of impurity particles from the kerosene containing impurity particles. The test results show that the separation efficiency increased by more than 32%.

**Embodiment 3**

The porous material of the present embodiment is porous tantalum and has two levels pore structure, classified by the material pore size. The pores within each level and the pores at different levels are three-dimensionally connected, with a total effective porosity of 80%. The pore size of the large pores is $400\mu m$-$600\mu m$, with pores having an average pore diameter of $30\mu m$ on the cavity walls. When the medium is New Zealand white rabbit blood, the capillary force is up to 2190Pa at 20 , calculated by the device shown in FIG.1 using formula (2). Using the same materials and processes of the preparation of the porous tantalum to prepare a same plane with the same state as cavity walls of the porous tantalum. According to the test method and conditions of Embodiment 1, the contact angle of the plane with New Zealand white rabbit blood is 70°. New Zealand rabbit blood has good infiltration to the surface of the material. The material can be used as a bone implant material for repairing femur defect caused by the lesion in the femur tissue repair of New Zealand white rabbit's test.

Each animal organ, every tissue cell is provided by the microcirculation of oxygen, nourishment, transfer of energy, exchange of information, removal of carbon dioxide and metabolic waste. Once the microcirculation is in disorder, its corresponding tissue system or internal organs will be affected and can not perform normal function, which can easily to lead to aging, immune disorders and diseases. At this time, if this kind of microcirculation system can be provided as a power source, the blood and tissue fluid can flowed and exchange properly, which will overcome the microcirculation obstacle.

In the embodiment, due to the lesion of the original femur caused by poor microcirculation around the lesion, the porous tantalum is implanted into the rabbit femur as a bone implant material, due to the strong capillary force, the porous tantalum acts as a microcirculation power source, promoting the blood and tissue fluid exchange, accelerating the formation of microcirculation blood vessels, so as to promote smooth flow of microcirculation, promote the growth of bone cells, and accelerate the repair of bone tissues.

After 12 weeks of implantation, the examination results showed that the new bone grows into porous tantalum and closely combines with the porous tantalum. The surrounding tissue grows well, the microvascular is full as web-like, and the implantation effect is good.

**Claims**

1.    A porous material, **characterized in that**: the porous material comprises a pore cavities and cavity walls surrounding the pore cavities, wherein the pore cavities of the porous material are three-dimensionally interconnected; a capillary force of the porous material is 5 Pa or more; and a contact angle between a surface of the cavity wall of the porous material and a liquid phase material circulating therein is less than 90°; the porous material is applied as a microcirculation power source.

2.    Use of porous material according to claim 1, in a circulation system as a microcirculation power source for providing a power source of material exchange.

3.    Use of porous material according to claim 1, in a separation system as a microcirculation power source for providing a power source of material separation and movement.

4.    Use of porous material according to claim 1, c in a medical implant system as a microcirculation power source for providing a power source of tissue cell growth.

Fig.1

Fig.2

# INTERNATIONAL SEARCH REPORT

| International application No. |
|---|
| **PCT/CN2016/098414** |

## A. CLASSIFICATION OF SUBJECT MATTER

A61L 27/56 (2006.01) i; A61L 27/04 (2006.01) i; A61L 27/08 (2006.01) i

According to International Patent Classification (IPC) or to both national classification and IPC

## B. FIELDS SEARCHED

Minimum documentation searched (classification system followed by classification symbols)

A61L

Documentation searched other than minimum documentation to the extent that such documents are included in the fields searched

Electronic data base consulted during the international search (name of data base and, where practicable, search terms used)

WPI, EPODOC, CNKI, CNPAT, PubMed, GOOGLE Scholar, ISI Web of Knowledge: CHONGQING RUNZE PHARMACEUTICAL, porous, prosthetic material, implant material, three-dimensional connectivity; YE, Lei; biological porous, tantalum, medical implant material, 3d-interconnected

## C. DOCUMENTS CONSIDERED TO BE RELEVANT

| Category* | Citation of document, with indication, where appropriate, of the relevant passages | Relevant to claim No. |
|---|---|---|
| X | CN 102796907 A (CHONGQING RUNZE PHARMACEUTICAL CO., LTD.), 28 November 2012 (28.11.2012), description, paragraphs 0005-0009, and embodiments 2-7 | 1-4 |
| X | CN 103463674 A (CHONGQING RUNZE PHARMACEUTICAL CO., LTD.), 25 December 2013 (25.12.2013), description, paragraphs 0008-0012, and embodiments 7-12 | 1-4 |
| X | CN 102475903 A (CHONGQING RUNZE MEDICAL INSTRUMENT CO., LTD.), 30 May 2012 (30.05.2012), description, paragraphs 0007-0008 and 0010-0012 | 1-4 |
| X | US 5282861 A (ULTRAMET), 01 February 1994 (01.02.1994), claims 1-18, and figures 1-4 | 1-4 |

☐ Further documents are listed in the continuation of Box C.   ☒ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 24 October 2016 (24.10.2016) | **15 November 2016 (15.11.2016)** |

| Name and mailing address of the ISA/CN: State Intellectual Property Office of the P. R. China No. 6, Xitucheng Road, Jimenqiao Haidian District, Beijing 100088, China Facsimile No.: (86-10) 62019451 | Authorized officer **TU, Haihua** Telephone No.: (86-10) **62413749** |
|---|---|

Form PCT/ISA/210 (second sheet) (July 2009)

## INTERNATIONAL SEARCH REPORT
### Information on patent family members

International application No.

**PCT/CN2016/098414**

| Patent Documents referred in the Report | Publication Date | Patent Family | Publication Date |
|---|---|---|---|
| CN 102796907 A | 28 November 2012 | WO 2013113250 A1 | 08 August 2013 |
| | | CN 102796907 B | 10 December 2014 |
| CN 103463674 A | 25 December 2013 | CN 103463674 B | 20 May 2015 |
| CN 102475903 A | 30 May 2012 | CN 102475903 B | 18 September 2013 |
| US 5282861 A | 01 February 1994 | EP 0560279 B1 | 14 June 2000 |
| | | JP H07255832 A | 09 October 1995 |
| | | JP 3445301 B2 | 08 September 2003 |
| | | DE 69328843 T2 | 02 November 2000 |
| | | EP 0560279 A1 | 15 September 1993 |
| | | ES 2148191 T3 | 16 October 2000 |
| | | DE 69328843 D1 | 20 July 2000 |

Form PCT/ISA/210 (patent family annex) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 1636876 A
- GB 525093 A